Europäisches Patentamt

European Patent Office

Office européen des brevets

(11) Publication number: **0 164 036**
**B1**

# (12) EUROPEAN PATENT SPECIFICATION

(45) Date of publication of patent specification: **02.11.88**

(21) Application number: **85106410.5**

(22) Date of filing: **24.05.85**

(51) Int. Cl.⁴: **A 61 K 35/60** // (A61K35/60, 31:375)

(54) Composition for accelerating recovery of function of hematopoietic organs and the use thereof.

(30) Priority: **26.05.84 JP 106971/84**

(43) Date of publication of application:
**11.12.85 Bulletin 85/50**

(45) Publication of the grant of the patent:
**02.11.88 Bulletin 88/44**

(84) Designated Contracting States:
**AT BE CH DE FR GB IT LI LU NL SE**

(56) References cited:

**CHEMICAL ABSTRACTS, vol. 30, nr. 21, November 10, 1936, column 7641 - 3; Columbus, Ohio, US I.A. MANVILLE et al.: "The effect of brewers'yeast on blood production."**

**CHEMICAL ABSTRACTS, vol. 66, nr. 2, January 9, 1967, page 833, ref. nr. 8675s; Columbus, Ohio, US O.I. BELOUSOVA: "Combined therapy of subacute radiation sickness in guinea pigs induced by chronic irradiation.".**

(73) Proprietor: **NISSAN CHEMICAL INDUSTRIES LTD.**
**3-7-1, Kanda Nishiki-cho**
**Chiyoda-ku Tokyo (JP)**

(72) Inventor: **Morishige, Fukumi**
**961, Takoe Miwamachi**
**Asakura-gun Fukuoka-ken (JP)**

(74) Representative: **Eitle, Werner, Dipl.-Ing. et al**
**Hoffmann, Eitle & Partner Patentanwälte**
**Arabellastrasse 4**
**D-8000 München 81 (DE)**

Courier Press, Leamington Spa, England.

# 0 164 036

**Description**

This invention relates to a composition for accelerating a function of a hematopoietic organ comprising a mixture of a milt extract and vitamin C.

For the purpose of accelerating recovery of physical strength of patients under treatment or after treatment, particularly of patients under or after treatment for inhibition nucleic acid metabolism of cancer cells, such as administration of anticancer agents and radiation, it is one of important factors to aim at recovery of a hematopoietic function of the bone marrow.

The hematopoietic function of cancer-bearing patients undertaking pharmacotherapy with anticancer agents or radiotherapy is reduced, and in particular, blood platelets and leucocytes in blood are significantly reduced in number. This reduction in hematopoietic function necessarily leads to hypofunction of biophylaxis. For this reason, recovery of a hematopoietic function of the bone marrow is contemplated.

To this effect, auxotherapy from a nutrological standpoint has been proposed for treatment of cancers in addition to surgical or chemotherapeutic treatment. For example, it has been considered useful for patients to take beer yeast having high nucleic acid contents as described, e.g., by Kimoto et al., *Kakusan no Eiyogaku*, 52, Nihon Bunshi Seigo Eiyogaku Kenkyu Zaidan (Aug. 25, 1983).

The above-described therapy aims at acceleration of recovery of tissue cells of patients under or after treatment by means of supplementation of nucleic acid synthetic ability of tissue cells through salvage synthesis (cf. page 34 of the above-cited reference). It further aims at selective utilization of the administered nucleic acids and/or the degradation products thereof, i.e., nucleotide, nucleoside and nucleic acid bases, by normal bone marrow cells, which have been partly damaged by the administration of anticancer agents and the like rather than by cancer cells by virtue of the salvage synthesis (cf. page 38 of the above-cited reference).

As set forth above, administration of drugs having high nucleic acid contents in combination with pharmacotherapy or radiotherapy is considered effective in the treatment of patients with cancers.

However, when such drugs having high nucleic acid contents are given to patients suffering from hyperoxia, there is the danger that the uric acid level in blood further increases, resulting in induction of complications, such as gouty arthritis, tophus, renal disorders, urolithiasis and arterisclerosis.

Therefore, there is a demand for a drug having a high nucleic acid content for recovering a hematopoietic function of the bone marrow to thereby restore physical strength of patients without accompanying a rise of the uric acid level in blood that possibly causes the above-described complications.

There is a further demand for a nutritive source which enables patients under treatment or convalescents to easily recover physical strength through acceleration of recovery of the function of the hematopoietic organs.

Accordingly, an object of this invention is to provide a medical composition for accelerating recovery of physical strength of patients under or after treatment.

Another object of this invention is to provide a medical composition which can prevent reduction of platelets and leucocytes in blood caused by the administration of anticancer agents or by radiotherapy for the inhibition of nucleic acid metabolism of cancer cells, to thereby accelerate recovery of a hematopoietic function of the bone marrow.

The present inventor has found that a milt extract of fish has a high content of nucleic acids and the nucleic acids are present therein in the form of a combination with proteins together with a sugar portion and a mineral. It has also been found that functional recovery of the hematopoietic organ can satisfactorily be achieved and also the uric acid level in blood does not increase or rather decrease by orally administering a combination of such a milt extract with vitamin C and/or a pharmaceutically acceptable salt thereof, and, thus, the present invention has been completed.

The invention comprises a composition for accelerating a function of a hematopoietic organ, which comprises

(a) a mixture of a milt extract of fish having a weight ratio of nucleic acids to proteins of from 1.0:1.0 to 2.0:1.0; nucleic acid content of 25 to 50 wt%; a protein content of 25 to 50 wt%; a mineral content of 5 to 15 wt%; and a total nitrogen content of 13 to 20 wt.%; and is positive in ninhydrin reaction and

(b) vitamin C and/or a pharmaceutically acceptable salt thereof in a weight ratio of (a):(b) of from 1:1 to 1:100.

Figure 1 shows results of measurement of blood uric acid levels at prescribed intervals in clinical cases (1) to (4). The patients had fasted and were fed with an artificial nutrient containing no nucleic acid and received nucleic acids alone or a powder preparation (nucleic acid plus sodium salt of vitamin C) according to the present invention.

Figures 2 and 3 each illustrates changes of leucocytes or blood platelets in number, respectively, when a composition of the present invention was administered to patients who had been healed with 5-fluorouracil, an anticancer agent.

Figure 4 shows changes of blood uric acid levels in the same cases as in Figures 2 and 3.

Figure 5 shows changes of leucocytes, blood platelets, erythrocytes, hemoglobins and hematocrit respectively, when a composition of the present invention was administered to an anemic patient.

The milt extract has high contents of nucleic acids, and these nucleic acids are present therein in the

2

form of nucleic acid-protein combination. The milt extract additionally contains sugars and inorganic matters. When the milt extract is mixed with vitamin C or its salt, these components all-inclusively exhibit a remarkable effect of accelerating recovery of a hematopoietic function of hematopoietic organs, particularly bone marrow. Despite of the high nucleic acid contents of the milt extract, the composition according to the present invention, when orally administered, suppresses an increase of the uric acid level in the blood serum of patients suffering from hyperoxia and, at the same time, prevents reduction of blood platelet and leucocytes as apparently demonstrated in the clinical examples hereinafter described. Therefore, the composition of the present invention is effective to recover physical strength of the patients without causing complications, such as gouty arthritis, tophus, renal disorders, urolithiasis or arteriosclerosis.

When the composition is orally administered, some part of the nucleic acids contained in the milt extract is degraded to nucleic acid bases via nucleotide and nucleoside by the time of absorption from an intestine as described in pages 27—28 of the Kimoto et al. reference. In some detail, orally administered nucleic acids are hydrolyzed with ribonuclease and deoxyribonuclease in pancreatic juice to form nucleotide, which is then degraded to nucleoside through release of phosphoric acid by the action of phosphatase. Nucleoside is further decomposed into bases and pentosugar by nucleosidase. The nucleic acids are assumed to be absorbed from the intestine in form of any of these degradation products.

Therefore, in the salvage synthesis of nucleic acids, it is easily anticipated that the effect of the orally administered nucleic acids can also be exerted by orally administered nucleotide, nucleoside and nucleic acid bases.

The milt extract which can be used in the composition according to the present invention can be obtained by extracting the milt of fish, particularly salmons, porgies, herrings, cods, sardines, swellfishes or bonitoes as follows. The milt of, for example, a salmon is filtered to remove any solid, such as the membrane, and to the filtrate is added a 0.14 mol/l aqueous solution of sodium chloride, followed by grinding and stirring to obtain a milky suspension. The suspension is centrifuged to separate the solid phase thus obtained. The supernatent liquor is then washed with a 0.14 mol/l aqueous solution of sodium chloride, followed by filtration. The above-described procedures from suspension through filtration were repeated two or three times. The finally obtained filtrate is washed with ethanol to remove ethanol-soluble organic matters and water, and dried under reduced pressure to obtain the desired milt extract.

The milt extract thus obtained from a salmon has the following chemical and physical properties:

| | |
|---|---|
| Weight ratio of nucleic acid to protein: | from 1.0:1.0 to 2.0:1.0 |
| Nucleic acid content: | 25—50 wt% |
| Protein content: | 25—50 wt% |
| Mineral content: | 5—15 wt% |
| Total nitrogen content: | 13—20 wt% |
| Ninhydrin reaction: | positive |

Milt extract prepared from other fish, e.g., porgies, herrings, cods, sardines, swellfishes, bonitoes, saurels or trouts have substantially the same chemical and physical properties as shown above.

Thus, the milt extract to be used in the present invention can be regarded as a nucleic acid-rich nucleic acid-protein combination.

Vitamin C or its pharmaceutically acceptable salt which can be used as another component constituting the composition of the present invention includes synthetically prepared vitamin C and naturally-occurring vitamin C and their alkali metal salts, e.g. the sodium salt, the potassium salt or the calcium salt. The vitamin C and its salt may have a high purity up to 100%. They may also be juices of vegetables or fruits, e.g., a mandarin orange, a lemon or a grapefruit or concentrates thereof.

The composition according to the present invention is orally administered in any of solid, fluid and liquid dose forms. The solid preparations may solely comprise the mixture of the milt extract and purified or crude (unpurified) vitamin C. The solid dose form may be prepared by adding at least one pharmacologically acceptable binder (e.g., syrups, gum arabic, gelatin, sorbitol, tragacanth, polyvinylpyrrolidone), vehicles (e.g., lactose, sugar, corn starch, calcium phosphate, sorbitol, glycine), lubricants (e.g., magnesium stearate, talc, polyethylene glycol, silica), disintegrators (e.g., potato starch), seasonings (e.g., sweeteners, salt, flavors, amino acids, e.g., sodium glutaminate) to the mixture according to the present invention and formulating the mixture in a known manner into powders, granules, tablets or capsules.

The fluid or liquid preparations may solely comprise the mixture according to the present invention, or may be prepared by adding water and, if desired, seasonings (e.g., sweeteners, edible salt, flavors, amino acids, e.g., sodium glutaminate) to the mixture.

The dose level of the milt extract in the novel mixture of the present invention is in the range of from 0.1 to 10 g, and preferably from 0.2 to 5 g, per day for an adult, and the dosage of the mixture being in the range of from 1 to 50 g, and preferably from 10 to 40 g, per day for an adult in a single or 3 divided doses. The above-specified dosage may be varied depending on the age, body weight, and symptoms.

Since the composition according to the present invention markedly prevents reduction of blood platelets and leucocytes which is particularly observed in patients suffering from cancers due to continuous administration of anticancer agents and also inhibits an increase of the uric acid level in blood, it accelerates recovery of a hematopoietic function of the bone marrow without being accompanied by complications, such as gouty arthritis, tophus, renal disorders, urolithiasis or arteriosclerosis and thus makes a great contribution to recovery of physical strength of patients with cancer or anaemia.

Hence, the method comprising orally administering the composition of the present invention to patients, particularly under or after treatment for cancer, offers an excellent nutrological auxotherapy.

Further, the above-described method of using the composition of the present invention is also applicable to recovery of physical strength of convalenscents.

The present invention will now be illustrated in greater detail referring to the following examples.

Preparation Example

211 grams of a milt obtained from a salmon were ground and filtered to remove solid matter including the membrane. To the filtrate was added 1.5 liters of a 0.14 mol/l aqueous solution of sodium chloride, and the mixture was ground and stirred to form a milky suspension. The suspension was subjected to centrifugation, and the supernatant liquor was separated. To the solid phase thus obtained was added 1.5 liters of a 0.14 mol/l aqueous solution of sodium chloride for washing, followed by filtration. These procedures involving suspension, centrifugation, washing and filtration were repeated 2 to 3 times. The resulting filtrate was washed with ethanol to remove ethanol-soluble organic matters and water. The ethanol-insoluble matter was dried under reduced pressure to obtain a powderous milt extract.

The thus obtained milt extract was a pale grayish white powder and had the following chemical and physical properties:

| | |
|---|---|
| Weight ratio of nucleic acid to protein: | from 1.0:1.0 to 2.0:1.0 |
| Nucleic acid content: | 25—50 wt% |
| Protein content: | 25—50 wt% |
| Mineral content: | 5—15 wt% |
| Total nitrogen content: | 13—20 wt% |
| Ninhydrin reaction: | positive |

In the same manner as described above, milt extracts were obtained from fish of other kinds.

Formulation Example 1

| | |
|---|---|
| Milt extract as prepared in preparation Example | 3.0 g |
| Sodium salt of vitamin C | 6.0 g |
| Total: | 9.0 g |

The above ingredients each pulverized to a particle size of 350 µm or smaller were weighed and uniformly mixed in a V-type mixer to prepare powders.

Formulation Example 2

| | |
|---|---|
| Milt extract as prepared in preparation example | 3.0 g |
| Sodium salt of vitamin C | 10.0 g |
| Total: | 13.0 g |

The above ingredients each pulverized to a particle size of 350 µm or smaller were weighed and uniformly mixed in a V-type mixer to prepare powders.

Formulation Example 3

| | |
|---|---|
| Milt extract as prepared in preparation example | 8.5 g |
| Sodium salt of vitamin C | 42.5 g |
| Rose hip | 3.0 g |
| Calcium panthotenate | 1.0 g |
| Powderous sugar | 15 g |
| Lactose | 40 g |
| Total: | 100 g |

The above ingredients were weighed and uniformly mixed to prepare granules.

### Formulation Example 4

| | |
|---|---:|
| Milt extract as prepared in preparation example | 8.5 g |
| Sodium salt of vitamin C | 42.5 g |
| Powderous sugar | 9.86 g |
| Lactose | 25.0 g |
| Rose hip | 3.0 g |
| Additives (flavors, lubricants, etc.) | 11.14 g |
| | Total:   100 g |

The above ingredients were weighed and uniformly mixed in a V-type mixer. The resulting powder mixture was directly used to prepare tablets each weighing 1 g.

Clinical Example

Nucleic acids alone or the powder preparations as prepared in Formulation Examples 1 or 2 was administered to patients who had fasted only uptaking an artificial nutrient containing no nucleic acid, and the uric acid levels in blood were measured at prescribed intervals.

Case (1):

Administration of nucleic acids alone

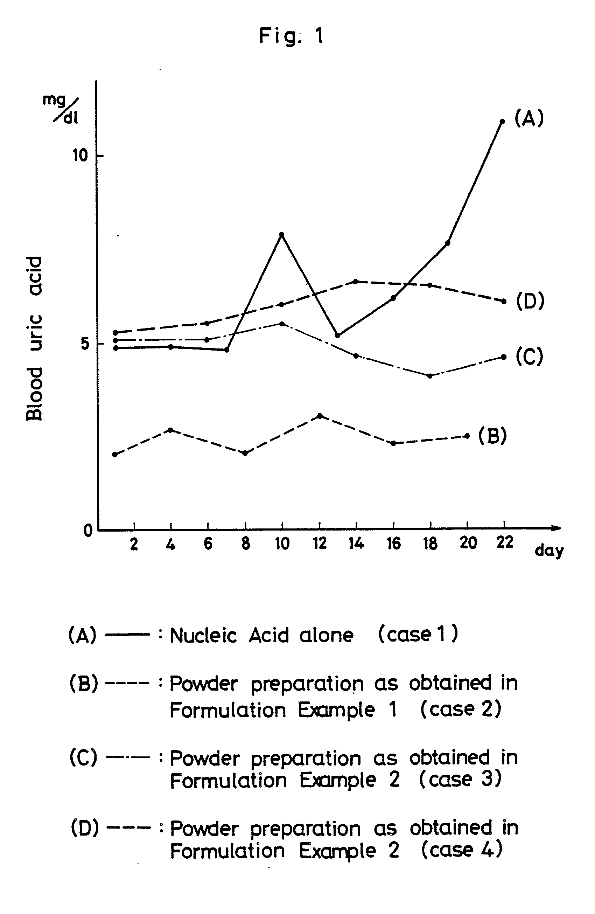

Only the milt extract as used in the powder preparation of Formulation Example 1 was administered to a 78-year-old male patient suffering from cerebral infarct and ischemic malum cordis at a dose of 3.0 g/day from the next day of the start of the determination of a blood uric acid level (i.e., the 2nd day in Figure 1) through the 22nd day, and the uric acid level in blood was determined every several days. The results obtained are shown in Figure 1.

Case (2):

Administration of powder preparation (milt extract plus sodium salt of vitamin C)

The powder preparation as prepared in Formulation Example 1 was administered to a 58-year-old female patient who suffered from ovarian cancer and cancerous peritonitis and who was maintained on Cysnoplatin (an anticancer agent) at a dose of 9.0 g per day from the next day of the start of the measurement of blood uric acid levels (i.e., the 2nd day in Figure 1) to the 20th day. The uric acid level in blood was measured every several days. The results obtained are shown in Figure 1.

Case (3):

Administration of powder preparation (milt extract plus sodium salt of vitamin C)

A daily dose of 13.0 g of the powder preparation as prepared in Formulation Example 2 was consecutively administered to a 55-year-old male patient suffering from cerebral infarct from the next day of the start of the measurement of blood uric acid levels (i.e., the 2nd day in Figure 1) to the 22nd day, and the uric acid level in blood was determined every several days. The results obtained are shown in Figure 1.

Case (4):

Administration of powder preparation (milt extract plus sodium salt of vitamin C)

A daily dose of 13.0 g of the powder preparation as prepared in Formulation Example 2 was administered to a 49-year-old male patient with primary liver cancer which had spread to the backbone and the left lung by metastasis from the first day of the start of the measurement of blood uric acid levels to the 22nd day. The uric acid level in blood was measured every several days, and the results obtained are shown in Figure 1.

As can be seen in Figure 1, the blood uric acid level in the case of administration of nucleic acids alone shows a rising tendency, whereas that each in the cases of administration of the powder preparations according to the present invention remains normal without any sign of rising.

Cases (5) to (8):

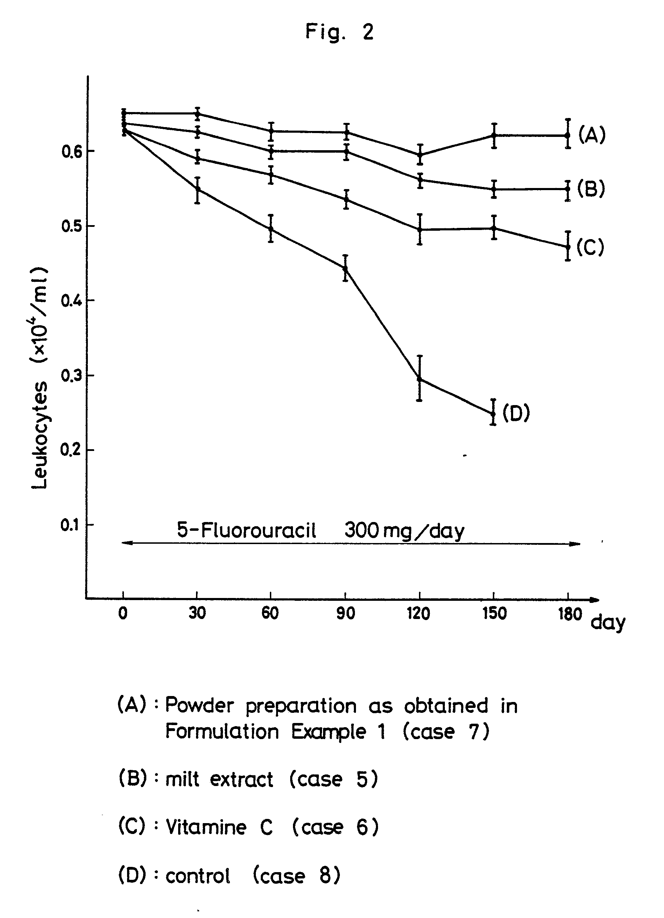

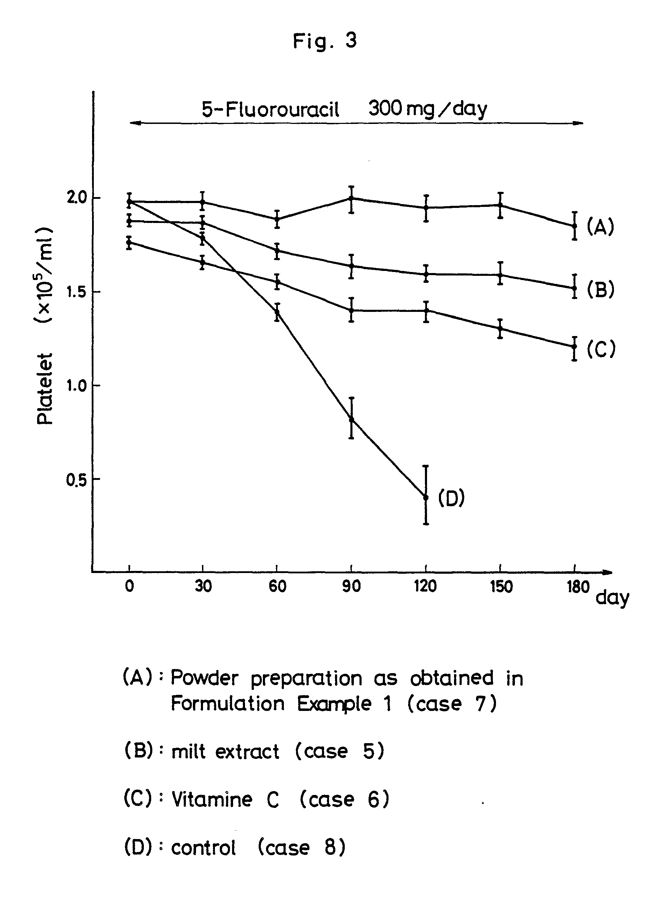

To each of three of four patients who had been healed with 5-fluorouracil (an anticancer agent), 500 mg a day, was administered a daily dose of 3 g of the milt extract originated in a salmon as used in Formulation Example 1 (Case (5)), a daily dose of 10 g of vitamin C (Case (6)), or a daily dose of 10 g of the powder preparation as obtained in Formulation Example 1 (Case (7)). As a control, no chemical was administered to one of the four patients (Case (8)). The leucocytes and platelets in blood were counted in each case every 30 days for a period of 180 days. The results obtained are shown in Figures 2 and 3, respectively.

As is apparent from Figures 2 and 3, it can be seen that the composition according to the present invention pronouncedly suppresses reduction of blood platelets and leucocytes which is attributed to administration of 5-fluorouracil and thus recovers the hematopoietic function of hematopoietic organs, particularly the bone marrow.

Cases (9) to (11):

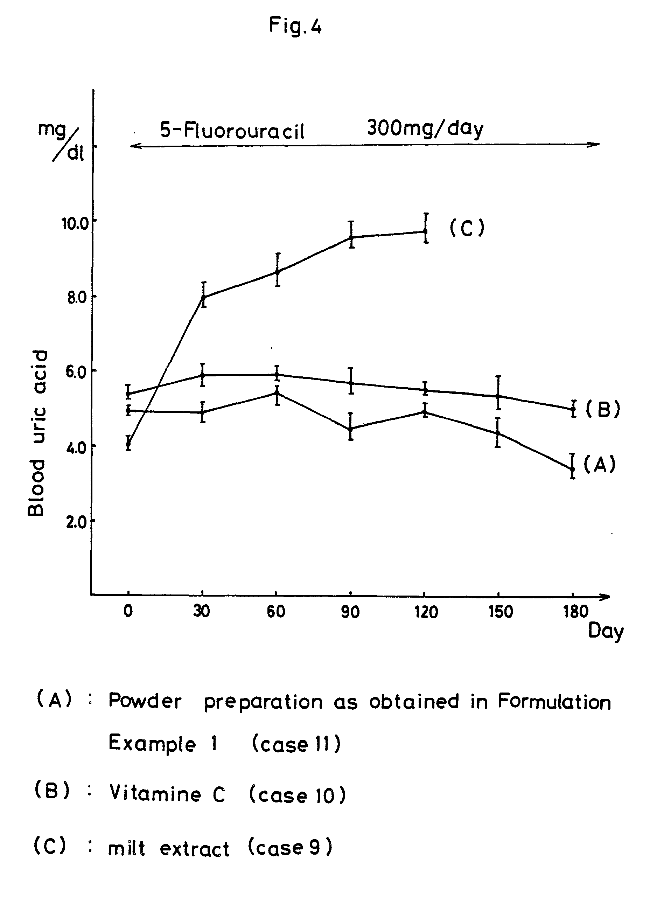

To each of three patients who had been healed with 5-fluorouracil, 300 mg a day, was administered a daily dose of 3 g of the milt extract as used in Formulation Example 1 (Case (9)), a daily dose of 10 g of vitamin C (Case (10)), or a daily dose of 10 g of the powder preparation as prepared in Formulation Example 1 (Case (11)). The uric acid level in blood in each patient was measured every 30 days for a period of 180 days. The results obtained are shown in Figure 4.

The results of Figure 4 proved that the composition of the present invention suppresses an increase of blood uric acid level and is, therefore, free from any danger to cause complications, such as gouty arthritis, tophus, renal disorders, urolithiasis or arteriosclerosis, in patients under treatment.

Case (21):

Administration example of the powder preparation to an anaemic patient

To a 70-year-old male patient suffering from was anaemic symptoms, continuously administered a daily dose of 10 g of the powder preparation of the Formulation Example 1.

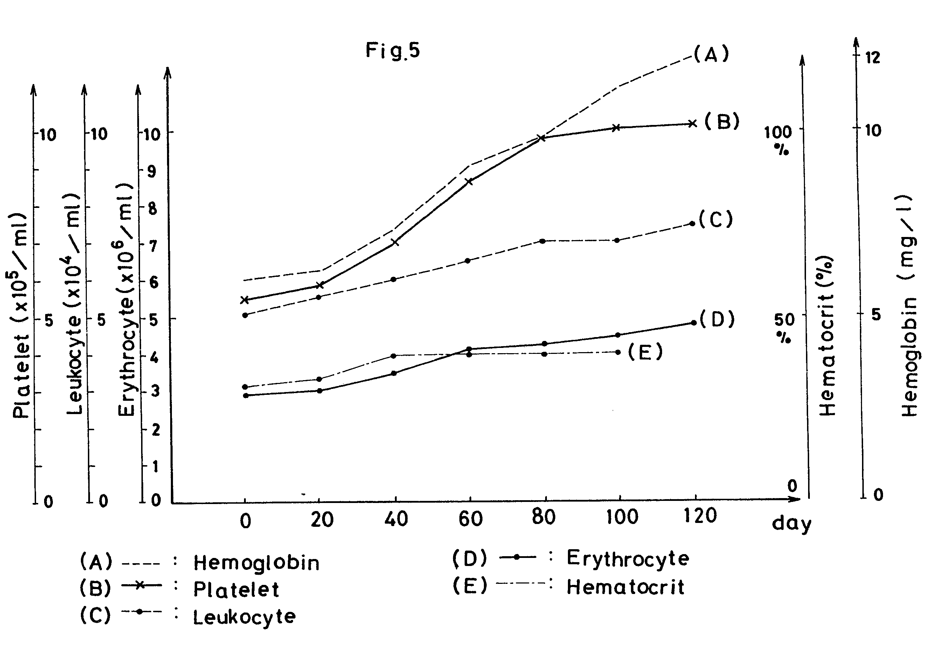

The results are shown in Figure 5.

As apparent from the changes shown in Figure 5, observation of peripheral blood was gradually recovered and normal observation was obtained after about three months.

Further, it has been revealed that the method according to the present invention which comprises orally administering the composition particularly to patients with cancers is effective for recovery of physical strength and is, therefore, an excellent nutrological auxotherapy.

**Claims for the Contracting States: BE, CH, DE, FR, GB, IT, LI, LU, SE**

1. A composition for accelerating a function of a hematopoietic organ, which comprises

(a) a mixture of a milt extract of fish having a weight ratio of nucleic acids to proteins of from 1.0:1.0 to 2.0:1.0; nucleic acid content of 25 to 50 wt%; a protein content of 25 to 50 wt%; a mineral content of 5 to 15 wt%; and a total nitrogen content of 13 to 20 wt.%; and is positive in ninhydrin reaction and

(b) vitamin C and/or a pharmaceutically acceptable salt thereof in a weight ratio of (a):(b) of from 1:1 to 1:100.

2. A composition as claimed in Claim 1, wherein the milt extract is originated in a salmon.

3. The use of a composition according to Claim 1 or 2 for the preparation of a composition for accelerating a function of the hematopoietic organ.

4. The use as claimed in Claim 3, wherein the composition is for administration to a convalenscent.

5. The use as claimed in Claim 3, wherein the composition is for administration to a patient with cancer.

6. The use as claimed in Claim 3, wherein the composition is for administration to a patient with anaemia.

7. The use as claimed in Claim 3, wherein the composition is for administration at a dose of from 1 to 15 g per day.

**Claims for the Contracting State: AT**

1. The use of (a) milt extract of fish having a weight ratio of nucleic acids to proteins of from 1.0:1.0 to 2.0:1.0; nucleic acid content of 25 to 50 wt%; a protein content of 25 to 50 wt%; a mineral content of 5 to 15 wt%; and a total nitrogen content of 13 to 20 wt.%; and is positive in ninhydrin reaction and (b) vitamin C and/or a pharmaceutically acceptable salt thereof in a weight ratio of (a):(b) of 1:1 to 1:100 for the preparation of a composition for accelerating a function of the hematopoietic organ.

2. The use as claimed in Claim 1, wherein the composition is for administration to a convalescent.

3. The use as claimed in Claim 1, wherein the composition is for administration to a patient with anaemia.

4. The use as claimed in any one of Claims 1, 2, 3 and 4, wherein the milt extract is originated in a salmon.

5. The use as claimed in Claim 1, wherein the composition is for administration to a patient with cancer.

6. The use as claimed in Claim 1, wherein the composition is for administration at a dose of from 1 to 15 g per day.

**Patentansprüche für die Vertragsstaaten: BE, CH, DE, FR, GB, IT, LI, LU, SE**

1. Zusammensetzung zur Beschleunigung der Funktion eines hämopoetischen Organes, umfassend

(a) eine Mischung von Milzextrakt von Fisch mit einem Gewichtsverhältni von Nukleinsäuren zu Proteinen von 1,0:1,0 bis 2,0:1,0; einem Nukleinsäuregehalt von 25 bis 50 Gew.-%; einem Proteingehalt von 25 bis 50 Gew.-%; einem Mineralgehalt von 5 bis 15 Gew.-% und einem Gesamtstickstoffgehalt von 13 bis 20 Gew.-%, wobei die Mischung eine positive Ninhydrinreaktion zeigt, und

(b) Vitamin C und/oder einem pharmazeutisch annehmbaren Salz davon in einem Gewichtsverhältnis von (a):(b) von 1:1 bis 1:100.

2. Zusammensetzung gemäß Anspruch 1, in welcher das Milzextrakt von einem Lachs stammt.

**0 164 036**

3. Verwendung der Zusammensetzung gemäß Anspruch 1 oder 2 zur Herstellung einer Zusammensetzung für die Beschleunigung der Funktion eines hämopoetischen Organes.

4. Verwendung gemäß Anspruch 3, in welcher die Zusammensetzung zur Verabreichung an einen Rekonvalezenten beabsichtigt ist.

5. Verwendung gemäß Anspruch 3, in welcher die Zusammsetzung für die Verabreichung an einen Krebspatienten beabsichtigt ist.

6. Verwendung gemäß Anspruch 3, in welcher die Zusammensetzung für die Verabreichung an einen anämischen Patienten beabsichtigt ist.

7. Verwendung gemäß Anspruch 3, in welcher die Zusammensetzung zur Verabreichung in einer Dosis von 1 bis 15 g pro Tag beabsichtigt ist.

**Patentansprüche für den Vertragsstaat: AT**

1. Verwendung von (a) Milzextrakt von Fisch mit einem Gewichtsverhältnis von Nukleinsäure zu Protein von 1,0:1,0 bis 2,0:1,0; einem Nukleinsäuregehalt von 25 bis 50 Gew.-%; einem Proteingehalt von 25 bis 50 Gew.-%; einem Mineralgehalt von 5 bis 15 Gew.-%, und einem Gesamtstickstoffgehalt von 13 bis 20 Gew.-%, wobei das Milzextrakt einen Gesamtstickstoffgehalt von 13 bis 20 Gew.-% hat, und positiv bei der Ninhydrinreaktion ist, und (b) Vitamin C und/oder eines pharmazeutisch annehmbaren Salzes davon in einem Gewichtsverhältnis von (a):(b) von 1:1 bis 1:100 zur Herstellung einer Zusammensetzung zum Beschleunigen der Funktion des hämopoetischen Organes ist.

2. Verwendung gemäß Anspruch 1, in welcher die Zusammensetzung für die Verabreichung an einen Rekonvalezenten bestimmt ist.

3. Verwendung gemäß Anspruch 1, in welcher die Zusammensetzung für die Verabreichung an einen anämischen Patienten bestimmt ist.

4. Verwendung gemäß Anspruch 1, in welcher die Zusammensetzung für die Verabreichung an einen Krebspatienten bestimmt ist.

5. Verwendung gemäß einem der Ansprüche 1, 2, 3 und 4, in welcher das Extrakt von einem Lachs stammt.

6. Verwendung gemäß Anspruch 1, in welcher die Zusammensetzung für die Verabreichung in einer Dosis von 1 bis 15 g pro Tag bestimmt ist.

**Revendications pour les Etats Contractants: BE, CH, DE, FR, GB, IT, LI, LU, SE**

1. Une composition pour accélérer une fonction d'un organe hématopoïétique qui comprend

(a) un mélange d'un extrait de laitance de poisson ayant un rapport pondéral des acides nucléiques aux protéines de 1,0/1,0 à 2,0/1,0; une teneur en acide nucléique de 25 à 50% en poids; une teneur en protéine de 25 à 50% en poids; une teneur en substances minérales de 5 à 15% en poids; et une teneur en azote total de 13 à 20% en poids; et donne une réaction positive avec la ninhydrine et

(b) de la vitamine C et/ou un sel convenant en pharmacie de celle-ci dans un rapport pondéral de (a)/(b) de 1/1 à 1/100.

2. Une composition comme revendiquée dans la revendication 1, dans laquelle l'extrait de laitance provient d'un saumon.

3. L'utilisation d'une composition selon la revendication 1 ou 2 pour la préparation d'une composition pour accélérer une fonction de l'organe hématopoïétique.

4. L'utilisation comme revendiqué dans la revendication 3, dans lequelle la composition est pour l'administration à un convalenscent.

5. L'utilisation comme revendiqué dans la revendication 3, dans laquelle la composition est pour l'administration à un patient atteint de cancer.

6. L'utilisation comme revendiqué dans la revendication 3, dans laquelle la composition est pour l'administration à un patient atteint d'anémie.

7. L'utilisation comme revendiqué dans la revendication 3, dans laquelle la composition est pour l'administration à une dose de 1 à 15 g par jour.

**Revendications pour l'Etat Contractant: AT**

1. L'utilisation de (a) un extrait de laitance de poisson ayant un rapport pondéral des acides nucléiques aux protéines de 1,0/1,0 à 2,0/1,0; une teneur en acide nucléique de 25 à 50% en poids; une teneur en protéine de 25 à 50% en poids; une teneur en substances minérales de 5 à 15% en poids; et une teneur en azote total de 13 à 20% en poids; et donne une réaction positive avec la ninhydrine et (b) de la vitamine C et/ou un sel convenant en pharmacie de celle-ci dans un rapport pondéral de (a)/(b) de 1/1 à 1/100 pour la préparation d'une composition pour accélérer une fonction de l'organe hématopoïétique.

2. L'utilisation comme revendiqué dans la revendication 1, dans laquelle la composition est pour l'administration à un convalescent.

3. L'utilisation comme revendiqué dans la revendication 1, dans laquelle la composition est pour l'administration à un patient atteint d'anémie.

7

4. L'utilisation comme revendiqué dans l'une quelconque des revendications 1, 2, 3 et 4 dans laquelle l'extrait de laitance provient d'un saumon.

5. L'utilisation comme revendiqué dans la revendication 1, dans laquelle la composition est pour l'administration à un patient atteint de cancer.

6. L'utilisation comme revendiqué dans la revendication 1, dans laquelle la composition est pour l'administration à une dose de 1 à 15 g pour jour.

Fig. 1

(A) ——— : Nucleic Acid alone　(case 1)

(B) ----- : Powder preparation as obtained in
　　　　　Formulation Example 1　(case 2)

(C) —·— : Powder preparation as obtained in
　　　　　Formulation Example 2　(case 3)

(D) --- : Powder preparation as obtained in
　　　　　Formulation Example 2　(case 4)

1

Fig. 2

(A) : Powder preparation as obtained in
Formulation Example 1 (case 7)

(B) : milt extract (case 5)

(C) : Vitamine C (case 6)

(D) : control (case 8)

## Fig. 3

(A) : Powder preparation as obtained in Formulation Example 1 (case 7)

(B) : milt extract (case 5)

(C) : Vitamine C (case 6)

(D) : control (case 8)

Fig. 4

(A) : Powder preparation as obtained in Formulation Example 1 (case 11)

(B) : Vitamine C (case 10)

(C) : milt extract (case 9)

Fig.5

(A) ----- : Hemoglobin     (D) —•— : Erythrocyte

(B) —×— : Platelet         (E) —•—•— : Hematocrit

(C) —•—•— : Leukocyte

0 164 036